# EUROPEAN PATENT APPLICATION

(11) **EP 4 042 962 A1**
(43) Date of publication of application: **17.08.2022**
(21) Application number: 22156431.3
(22) Date of filing: 11.02.2022
(51) Int. Cl.: A61B 18/14, H01R 24/00

(54) **CONTINUOUS ROTATION CABLE FOR SURGICAL INSTRUMENT**

(30) Priority: 12.02.2021 US 202117174676
(71) Applicant: Covidien LP, Mansfield, MA 02048 (US)
(72) Inventor: GOODMAN, Kelley, D., Boulder, CO Colorado 80301 (US); KRASTINS, Craig, V., 80301, CO Colorado 80301 (US); RICH, Jennifer, L, Boulder, CO Colorado 80301 (US); PEPIA, Riley, A, Boulder, CO Colorado 80301 (US); SIMS, Grant, T., Boulder, CO Colorado 80301 (US); MERCIER, Daniel, W., Boulder, CO Colorado 80301 (US)
(74) Representative: Maschio, Antonio

(57) **Abstract**

A coupling for an electrosurgical cable of a surgical instrument includes insulative contact collars arranged in a stack-like manner, each contact collar having a flange supporting a contact ring thereon. The contact rings configured for electrical engagement with electrical leads of the electrosurgical cable. Contact wires configured to engage corresponding contact rings at one end and a jumper wire at the other end thereof, the jumper wires, in turn, couple to an activation switch of the surgical instrument. A locking bobbin envelops the contact collars to lock contact wires in secure engagement with the contact rings. The locking bobbin locks within the surgical instrument preventing the contact wires from rotating within the surgical instrument while permitting the contact collars, the contact rings and the electrosurgical cable to rotate relative to the surgical instrument.

## Description

### BACKGROUND

### Technical Field

The present disclosure relates to surgical instruments and, more particularly, to a surgical forceps having an cable configured for continuous rotation attached thereto for reducing tangling during surgical procedures.

### Description of Related Art

A surgical forceps is a plier-like instrument which relies on mechanical action between its jaws to grasp tissue. Electrosurgical forceps utilize both mechanical clamping action and electrical energy to treat tissue, e.g., coagulate, cauterize, and/or seal tissue. Typically, once tissue is treated, the surgeon has to accurately sever the treated tissue. Accordingly, many electrosurgical forceps have been designed which incorporate a knife configured to effectively sever tissue after treating the tissue.

Various types of surgical forceps utilize different types of energy modalities to coagulate, cauterize, transect or seal vessels. As a result, one or more cables are attached to the forceps to provide electrical energy thereto. In some instances these cables are long and tend to tangle as a result of the surgeon manipulating the forceps during a given surgical procedure.

### SUMMARY

As used herein, the term "distal" refers to the portion that is being described which is further from a user, while the term "proximal" refers to the portion that is being described which is closer to a user.

Provided in accordance with one aspect of the present disclosure is a coupling for an electrosurgical cable of a surgical instrument that includes a plurality of insulative contact collars arranged in a stack-like manner. Each of the plurality of contact collars includes a flange configured to support a corresponding plurality of contact rings thereon. The plurality of contact rings is configured for electrical engagement with a corresponding plurality of electrical leads disposed through an electrosurgical cable. A plurality of contact wires is included wherein each contact wire is configured to engage a corresponding contact ring at one end thereof and a jumper wire at another end thereof. The jumper wires are adapted to electrical couple to an activation switch of the surgical instrument.

Aa locking bobbin is configured to at least partially envelop the plurality of insulative contact collars and lock the plurality of contact wires in secure electrical engagement with the plurality of contact rings. The locking bobbin includes one or more mechanical interfaces that cooperates with one or more corresponding mechanical interfaces disposed within the surgical instrument to secure the locking bobbin therein.

In aspects according to the present disclosure, the plurality of collars defines a passageway therethrough for receiving the electrical leads from the electrosurgical cable. In other aspects according to the present disclosure, the contact wires are U-shaped and include two opposing legs, the opposing legs of each contact wire configured to engage the opposing sides of each contact ring.

In aspects according to the present disclosure, the flanges of each contact collar of the plurality of contact collars is recessed relative to an outer peripheral surface of each contact collar of the plurality of contact collars such that, when electrically engaged with the corresponding plurality of contact wires, the plurality of contact wires lie flush with the outer peripheral surface of the plurality of contact collars.

In aspects according to the present disclosure, the locking bobbin includes a corresponding plurality of slots defined therein configured to receive the plurality of contact wires therethrough. In other aspects according to the present disclosure, each slot of the plurality of slots includes a recess configured to lock the respective contact wire therein. In still other aspects according to the present disclosure, each recess of each slot of the plurality of slots is configured to lock the respective contact wire therein upon movement of the locking bobbin relative to the plurality of contact collars from a first position to a second position.

In aspects according to the present disclosure, each lead of the plurality of electrical leads disposed through the electrosurgical cable is soldered to each corresponding contact ring. In other aspects according to the present disclosure, each contact wire of the plurality of contact wires is soldered to a corresponding jumper wire.

In aspects according to the present disclosure, each recess of each slot of the plurality of slots is angled relative to each slot of the plurality of slots to lock the respective contact wire therein upon movement of the locking bobbin relative to the plurality of contact collars from a first position to a second position.

In aspects according to the present disclosure, the locking bobbin includes at least one elongated flange that cooperates with a corresponding elongated channel disposed within the surgical instrument to secure the locking bobbin therein

Provided in accordance with one aspect of the present disclosure is a surgical instrument that includes an continuous rotation coupling (CRC) having: a plurality of insulative contact collars arranged in a stack-like manner, each of the plurality of contact collars including a flange configured to support a corresponding plurality of contact rings thereon, the plurality of contact rings configured for electrical engagement with a corresponding plurality of electrical leads disposed through an electrosurgical cable; and a plurality of contact wires, each contact wire configured to engage a corresponding contact ring at one end thereof and a jumper wire at another end thereof, the jumper wires adapted to electrical couple to an activation switch of a surgical instrument.

A locking bobbin is configured to at least partially envelop the CRC to lock the plurality of contact wires in secure electrical engagement with the plurality of contact rings. The locking bobbin includes one or more mechanical interfaces that cooperates with one or more corresponding mechanical interfaces disposed within the surgical instrument to secure the locking bobbin therein. The locking bobbin prevents the plurality of contact wires from rotating within the surgical instrument while permitting the plurality of contact collars, the plurality of contact rings and the electrosurgical cable to rotate relative thereto. A strain relief is disposed about the electrical cable. The strain relief is configured to seat within a strain cavity defined within a proximal flange of the surgical instrument. The strain relief is configured to allow rotation of the electrosurgical cable relative to the surgical instrument but prevent translation of the electrosurgical cable relative to the surgical instrument.

In aspects according to the present disclosure, the strain relief is crimped to the electrosurgical cable. In other aspects according to the present disclosure, the plurality of collars defines a passageway therethrough for receiving the electrical leads from the electrosurgical cable.

In aspects according to the present disclosure, the contact wires are U-shaped and include two opposing legs, the opposing legs of each contact wire configured to engage the opposing sides of each contact ring. In other aspects according to the present disclosure, the flanges of each contact collar of the plurality of contact collars is recessed relative to an outer peripheral surface of each contact collar of the plurality of contact collars such that, when electrically engaged with the corresponding plurality of contact wires, the plurality of contact wires lie flush with the outer peripheral surface of the plurality of contact collars.

In aspects according to the present disclosure, the locking bobbin includes a corresponding plurality of slots defined therein configured to receive the plurality of contact wires therethrough. In other aspects according to the present disclosure, each slot of the plurality of slots includes a recess configured to lock the respective contact wire therein. In still other aspects according to the present disclosure, each recess of each slot of the plurality of slots is configured to lock the respective contact wire therein upon movement of the locking bobbin relative to the plurality of contact collars from a first position to a second position.

In aspects according to the present disclosure, each lead of the plurality of electrical leads disposed through the electrosurgical cable is soldered to each corresponding contact ring. In other aspects according to the present disclosure, each contact wire of the plurality of contact wires is soldered to a corresponding jumper wire. In still other aspects according to the present disclosure, each recess of each slot of the plurality of slots is angled relative to each slot of the plurality of slots to lock the respective contact wire therein upon movement of the locking bobbin relative to the plurality of contact collars from a first position to a second position.

In aspects according to the present disclosure, the locking bobbin includes one or more elongated flanges that cooperates with a corresponding elongated channel disposed within the surgical instrument to secure the locking bobbin therein.

### BRIEF DESCRIPTION OF THE DRAWINGS

Various aspects of the present disclosure are described herein with reference to the drawings wherein like reference numerals identify similar or identical elements:
FIG. 1A is a side, perspective view of a forceps including opposing shaft members and an end effector assembly disposed at a distal end thereof according to an aspect of the present disclosure;
FIG. 1B is schematic diagram of a multi-modality surgical system including the forceps of FIG. 1A coupled to an electrosurgical generator and a foot switch connected the generator;
FIG. 2A is a side, perspective view of the forceps of FIG. 1A shown with continuous rotation coupling;
FIG. 2B is an enlarged, internal view of the continuous rotation coupling of FIG. 2A;
FIG. 3A is a greatly-enlarged internal view of a series of contact collars of the continuous rotation coupling of FIG. 2A shown without a locking bobbin disposed thereon;
FIG. 3B is a greatly-enlarged internal view of the continuous rotation coupling of FIG. 2A shown with the locking bobbin atop the contact collars;
FIGS. 4A and 4B are exploded and assembled views of the contact collar;
FIGS. 5A-5H are various views showing the assembly of the continuous rotation coupling of FIG. 2A;
FIG. 6 is a greatly-enlarged internal view of a strain relief ring for the continuous rotation coupling; and
FIGS. 7A and 7B are various view of an alternate locking bobbin for use with the continuous rotation coupling.

### DETAILED DESCRIPTION

Throughout the description, like reference numerals and letters indicate corresponding structure throughout the several views. Also, any particular feature(s) of a particular exemplary embodiment may be equally applied to any other exemplary embodiment(s) of this specification as suitable. In other words, features between the various exemplary embodiments described herein are interchangeable as suitable, and not exclusive.

Embodiments of the disclosure include systems, devices, and methods to control tissue temperature at a tissue treatment site during an electrosurgical procedure, as well as shrinking, coagulating, cutting, and sealing tissue against blood and other fluid loss, for example, by shrinking the lumens of blood vessels (e.g., arteries or veins). In some embodiments, the devices may be configured, due to the narrow electrode size, to fit through a trocar down to a size as small as 5 mm.

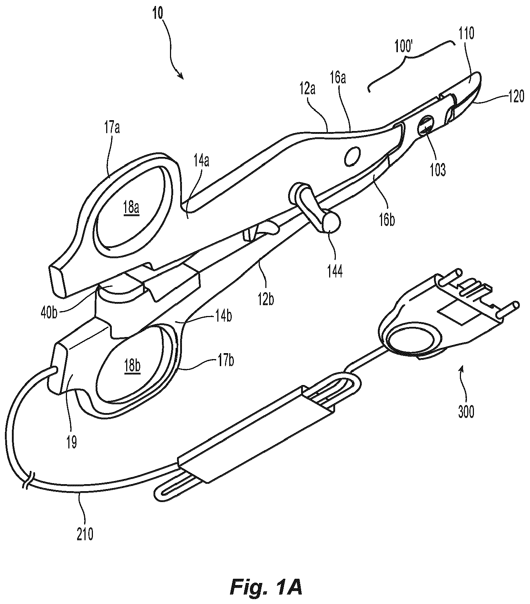

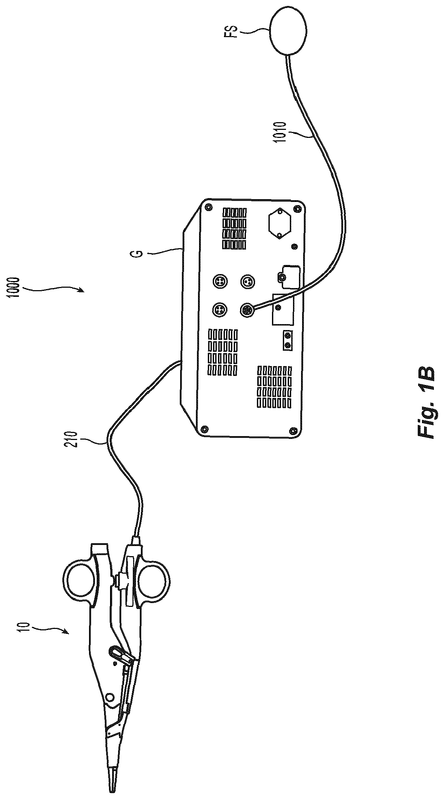

Referring now to FIGS. 1A and 1B, an open forceps 10 contemplated for use in connection with traditional open surgical procedures is shown. For the purposes herein, either an open instrument, e.g., forceps 10, or an endoscopic instrument (not shown) may be utilized in accordance with the present disclosure. Obviously, different electrical and mechanical connections and considerations apply to each particular type of instrument; however, the novel aspects with respect to the end effector assembly and its operating characteristics remain generally consistent with respect to both the open and endoscopic configurations.

With continued reference to FIGS. 1A and 1B, forceps 10 includes two elongated shafts 12a and 12b, each having a proximal end 14a and 14b, and a distal end 16a and 16b, respectively. Forceps 10 further includes an end effector assembly 100 attached to distal ends 16a and 16b of shafts 12a and 12b, respectively. End effector assembly 100 includes a pair of opposing jaw members 110 and 120 that are pivotably connected about a pivot 103. Each shaft 12a and 12b includes a handle 17a and 17b disposed at the proximal end 14a and 14b thereof. Each handle 17a and 17b defines a finger hole 18a and 18b therethrough for receiving a finger of the user. Finger holes 18a and 18b facilitate movement of the shaft members 12a and 12b relative to one another between a spaced-apart position and an approximated position, which, in turn, pivot jaw members 110 and 120 from an open position, wherein the jaw members 110 and 120 are disposed in spaced-apart relation relative to one another, to a closed position, wherein the jaw members 110 and 120 cooperate to grasp tissue therebetween.

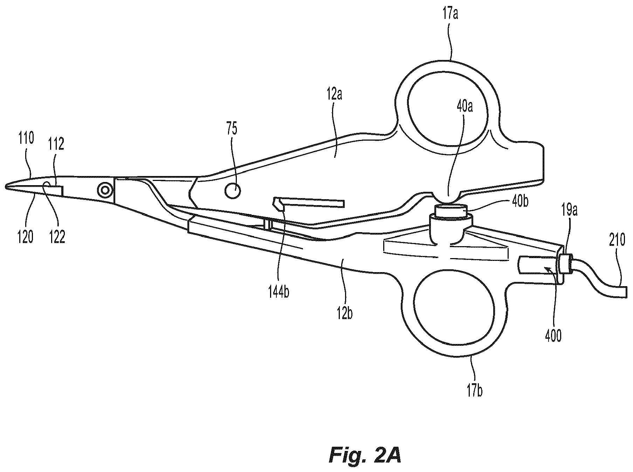

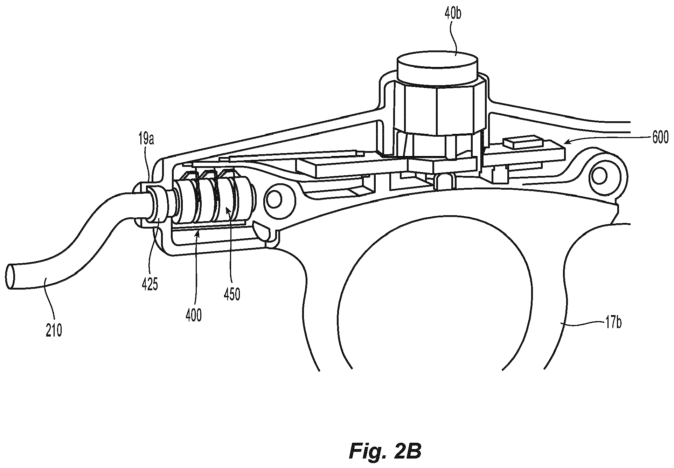

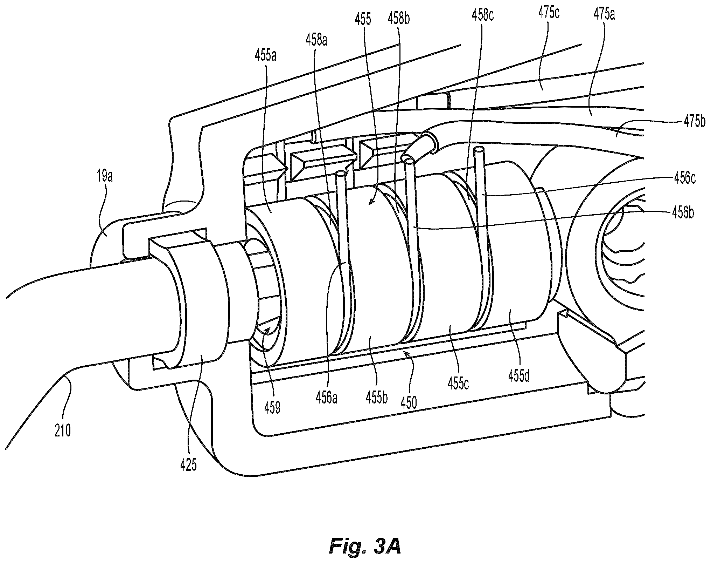

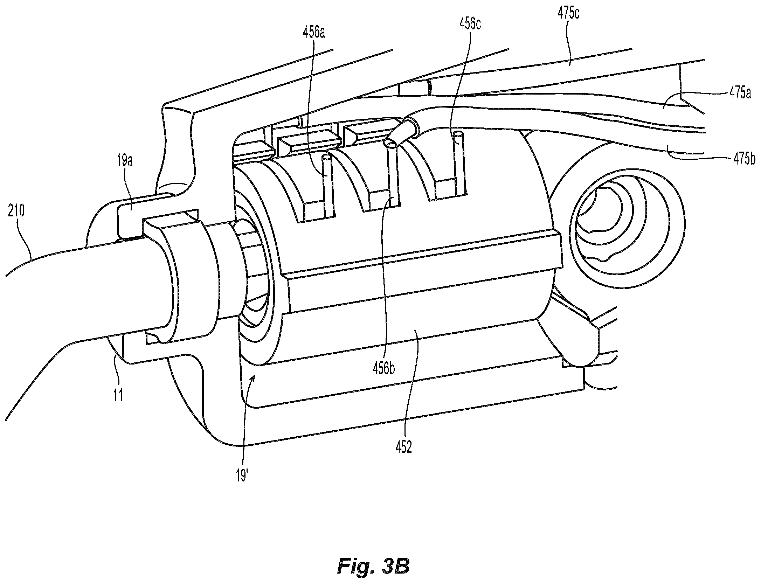

Continuing with reference to FIG. 1A, one of the shafts, e.g., shaft 12b, includes a proximal shaft connector 19 that is designed to connect the forceps 10 to a source of electrosurgical energy such as an electrosurgical generator G (FIG. 3). Proximal shaft connector 19 secures an electrosurgical cable 210 to forceps 10 such that the user may selectively apply electrosurgical energy to electrically-conductive plates 112 and 122 (see FIG 2) of jaw members 110 and 120, respectively. As discussed in more detail below, proximal shaft connector 19 includes a flange 19a that extends therefrom configured to receive an continuous rotation coupling 450 (FIG. 2B) therein.

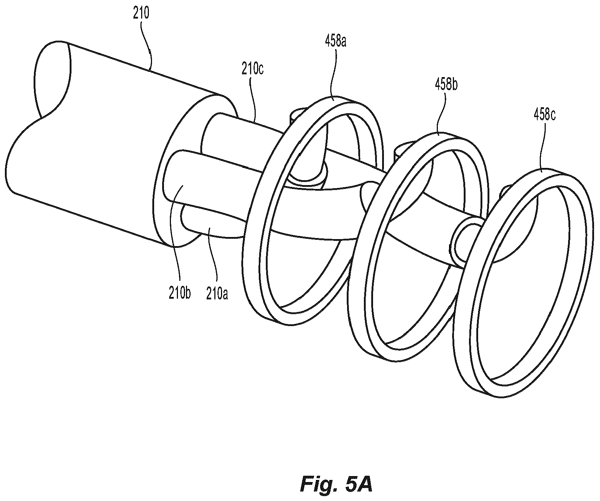

Cable 210 includes a plurality of wires 210a-210c (FIG. 5A) extending therethrough that have sufficient length to extend through one of the shaft members, e.g., shaft member 12b, in order to connect to a circuit board 600 which, in turn, when activated by activation switch 40b (See FIGS. 1A and 1B) provides electrical energy to the conductive plates 112, 122 of jaw members 110, 120, respectively, of end effector assembly 100 (or a second switch, e.g., footswitch FS (FIG. 1B)). Other types activation switches are also contemplated, e.g., finger switch, toggle switch, foot switch, etc. and may be configured for this purpose. Cable 210 operably connects to generator G via plug 300.

Activation switch 40b is disposed at proximal end 14b of shaft member 12b and extends therefrom towards shaft member 12a. A corresponding surface 40a is defined along shaft member 12a toward proximal end 14a thereof and is configured to actuate activation switch 40b. More specifically, upon approximation of shaft members 12a, 12b, e.g., when jaw members 110, 120 are moved to the closed position, activation switch 40b is moved into contact with, or in close proximity of surface 40a. Upon further approximation of shaft members 12a, 12b, e.g., upon application of a pre-determined closure force to jaw members 110, 120, activation switch 40b is advanced further into surface 40a to depress activation switch 40b. Activation switch 40b controls the supply of electrosurgical energy to jaw members 110, 120 such that, upon depression of activation switch 40b, electrosurgical energy is supplied to conductive surface 112 and/or conductive surface 122 of jaw members 110, 120, respectively, to seal tissue grasped therebetween. Electrical energy may be energy supplied through a proprietary Ligasure^{®} sealing algorithm LS owned by Covidien, LP (Medtronic). The switch 40b may be disposed on either shaft 12a, 12b.

Forceps 10 may further include a knife assembly (not shown) disposed within one of the shaft members, e.g., shaft member 12a and a knife channel (not shown) defined within one or both of jaw members 110, 120, respectively, to permit reciprocation of a knife (not shown) therethrough. Knife assembly includes a trigger 144a (FIG. 1A), 144b (FIG. 2A) coupled thereto that is actuatable to advance the knife from a retracted position within shaft member 12a, to an extended position wherein the knife extends into knife channels to divide tissue grasped between jaw members 110, 120. Other trigger assemblies are also contemplated.

Each jaw member 110, 120 of end effector assembly 100 may include a jaw frame having a proximal flange extending proximally therefrom that are engageable with one another to permit pivoting of jaw members 110, 120 relative to one another about a pivot 103 between the open position and the closed position upon movement of shaft members 12a, 12b relative to one another between the spaced-apart and approximated or closed positions. Proximal flanges of jaw members 110, 120 also connect jaw members 110, 120 to the respective shaft members 12b, 12a thereof, e.g., via welding, crimping or the like.

Jaw members 110, 120 may each further include an insulator (not shown) that is configured to receive a respective electrically-conductive tissue plate 112, 122, thereon and that is configured to electrically isolate the conductive plates 112, 122 from the remaining components of the respective jaw members 110, 120 (FIG. 2). Conductive plates 112, 122 are disposed in opposed relation relative to one another such that, upon movement of jaw members 110, 120 to the closed position, tissue is grasped between conductive plates 112, 122, respectively, thereof. Accordingly, in use, one or more modalities of electrosurgical energy may be supplied to one or both of conductive plates 112, 122 and conducted through tissue to treat tissue grasped therebetween. Knife may be advanced through knife channels of jaw members 110, 120 to cut tissue before, during or after treatment.

Turning to FIG. 1B, a schematic representation of a surgical system 1000 is shown and includes forceps 10, generator G and footswitch FS. In use, the forceps 10 connects to the generator G via plug 300 (See FIG. 1A). Activation of switch 40b of the forceps 10 provides electrical energy to the conducive plates 112, 122 utilizing a proprietary Ligasure^{®} sealing algorithm LS to seal tissue disposed between the jaw members 110, 120. The user squeezes handles 17a, 17b which, in turn, approximates the jaw members 110, 120. If it is desirous to seal tissue, the user fully approximates the handles 17a, 17b to activate activation switch 40b disposed therebetween. Once sealed, the user may actuate the knife assembly to cut the tissue disposed between the jaw members 110, 120.

As mentioned above, a footswitch FS may be operably coupled to the generator G via cable 1010. Upon actuation of the footswitch FS, electrical energy is transmitted to the conductive plates 112, 122 to treat tissue in a standard bipolar manner, e.g., for use with cauterizing tissue. The footswitch FS does not necessarily supply the necessary electrical energy to the tissue, but rather, is configured to send a control signal to the generator G to apply standard or known electrical, bipolar energy across the treat tissue. As can be appreciated, this configuration eliminates electrical energy flowing through the footswitch FS which can have negative effects thereon. Similarly, if the activation switch 40b is actuated upon full approximation of the jaw members 110, 120, a control signal is sent to the generator G to apply electrical energy across the tissue utilizing the Ligasure^{®} sealing algorithm LS.

Many iterations of the Ligasure^{®} sealing algorithm LS have been developed over the years and, as such, when using the term Ligasure^{®} sealing algorithm LS, all of these various iterations are envisioned. Details relating to some of the iterations of the Ligasure^{®} sealing algorithm LS are disclosed in U.S. Patent Nos. 8,920,421, 8,216,223, 6,398,779, 7,901,400, 7,972,328 the entire contents of each of which being incorporated by reference herein

When switch 40b is depressed, the generator G recognizes a voltage drop across the leads 210b and 210*c* which initiates activation of the generator G to supply a first electrical potential to jaw member 110 and a second electrical potential to jaw member 120 pursuant to the Ligasure^{®} algorithm LS. In this fashion, switch 40b acts more like a control circuit and is protected or removed from the actual current loop which supplies electrical energy to the jaw members 110 and 120. This reduces the chances of electrical failure of the switch 40b due to high current loads during activation. As mentioned above, footswitch FS also operates in a similar manner, i.e., upon activation of the footswitch FS, the generator G recognizes a voltage drop across the leads 210a, 210b which, in turn, signals the generator G to initiate electrosurgical activation of bipolar energy the jaw members 110 and 120.

Various tactile, audible and/or visual displays or alarms may be utilized to inform or confirm to the user that the proper or desired energy modality is being utilized. In other embodiments, alarms may be utilized to address concerns relating to energy delivery or switch priority concerns.

FIGS. 2A-3B show various views of an continuous rotation coupling (CRC) 450 operably associated within the flange 19a of the proximal shaft connector 19 configured to reduce tangling of the cable 210 during forceps use. More particularly, CRC 450 is configured to seat within a cavity 19' defined within the proximal shaft connector 19 in a fashion to facilitate rotational freedom of the cable 210 without straining the electrical leads 210a-210c therein. Further, the CRC 450 includes a strain relief 425 that is configured to prevent relative movement between the cable 210 and the CRC 450 reducing tension on the electrical connections.

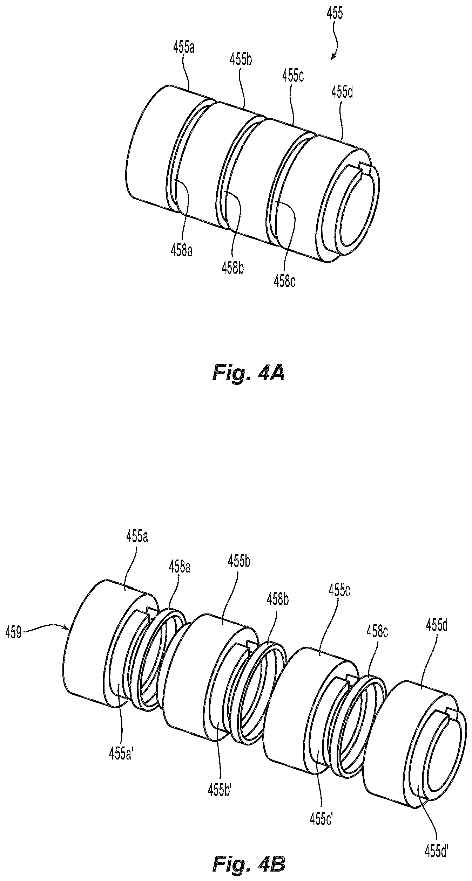

CRC 450 includes a series of contact collars 455a-455d that are arranged in a stack-like manner about a series of contact rings 458a-458c disposed between each adjacent collar, e.g., 455a and 455b (FIG. 4B). The collars 455a-455d include respective support flanges 455a'-455d' configured to support each respective ring 458a-458c thereon. The stacked collar assembly 455 is shown in FIG. 4A. The rings 458a-458c are made from electrically conductive material while the collars 455a-455d are made from an insulative material to reduce shorting between leads 210a-210c. The plurality of stacked collars 455a-455d collectively form a passageway 459 for receiving the electrical leads from cable 210 therethrough (FIGS. 3A and 4B). The distal-most contact collar 455d may be flush and not include a support flange. The support flanges 455a'-455d' are recessed to support the contact rings 458a-458c thereon.

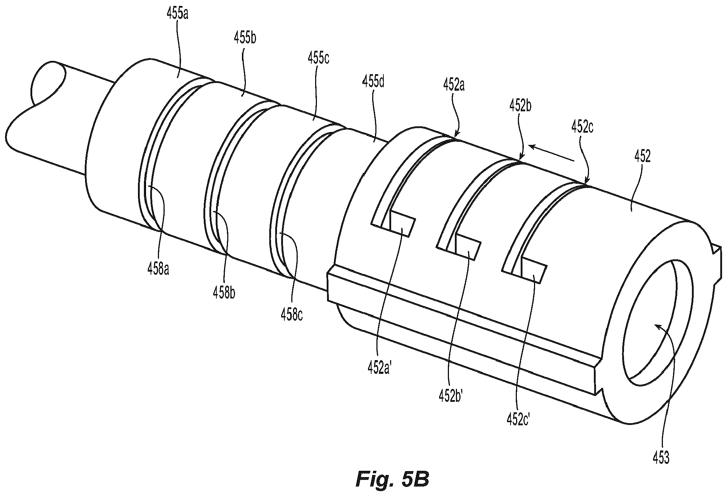

FIGS. 5A-5H show the various steps involved with the assembly of the CRC 450. Initially, leads 210a-210c are soldered or otherwise affixed to the internal peripheral surface of the contact rings 458a-458c of the CRC 450 (FIG. 5A). As can be appreciated, the collars 455a-455d may be separated to facilitate this purpose and, once the leads 210a-210c are soldered, thereafter stacked accordingly to secure the rings 458a-458c into the CRC 450. Once the collars 455a-455d are stacked and the rings 458a-458c secured with leads 210a-210c, a locking bobbin 452 is engaged atop the CRC 450 in sliding engagement (or snap-fit or otherwise) therewith (FIG. 5B).

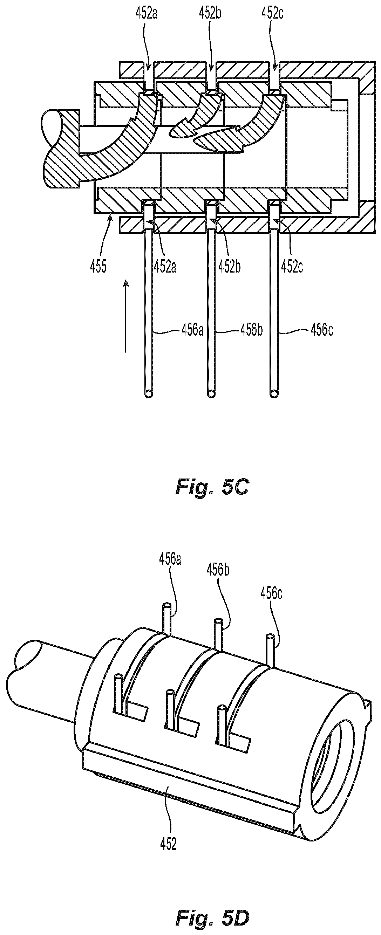

Bobbin 452 includes a corresponding series of slots defined in both an upper surface and lower surface, e.g., slots 452a-452c in the upper surface thereof and slots 453a-453c defined in the bottom surface thereof, that are each configured to receive a corresponding contact wire 456a-456c therein. Contact wires 456a-456c are generally U-shaped and include opposing legs that extend upwardly therefrom and into the slots 453a-453c defined in the bottom surface of bobbin 452 into contact with opposing sides of respective contact rings 458a-458c of the CRC 450 and out through slots 452a-452c in the upper surface of bobbin 452 (FIGS. 5C - 5D). In this first position, the contact rings 458a-458c disposed atop flanges 455a'-455c' of the contact collars 455a-455c (no contact ring is disposed atop flange 455d') align with respective slots 452a-452c and 453a-453c of the locking bobbin 452 so that the opposing legs may extend therethrough (FIG. 5C and 5D).

Contact wires 456a-456c may be "J" shaped or be configured as "pogo-pins" which are configured to electrically engage respective contact rings 458a458c. Moreover, the U-shaped contact wires 456a-456c may include loading bends, pinch points or be pre-loaded to facilitate secure engagement with contact rings 458a-458c

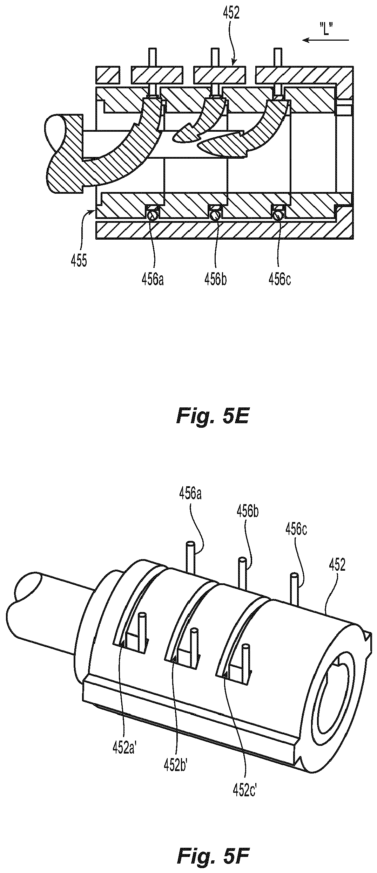

Once the wires 456a-456c are engaged through both upper and lower slots 452a-452c and 453a-453c respectively, the bobbin 452 is moved relative to the CRC 450, e.g., slid proximally in direction "L", to lock the contact wires 456a-456c in place in electrical engagement with the CRC 450 (FIGS. 5E and 5F). Upon proximal movement, the contact wires 456a-456c engage locking recesses 452a'-452c' defined within the corresponding slots 452a-452c of bobbin 452. In this second position, the slots 452a-452c and 453a-453c of the locking bobbin 452 are misaligned thereby preventing the contact wires 456a-456c from disengaging (FIG. 5E and 5F).

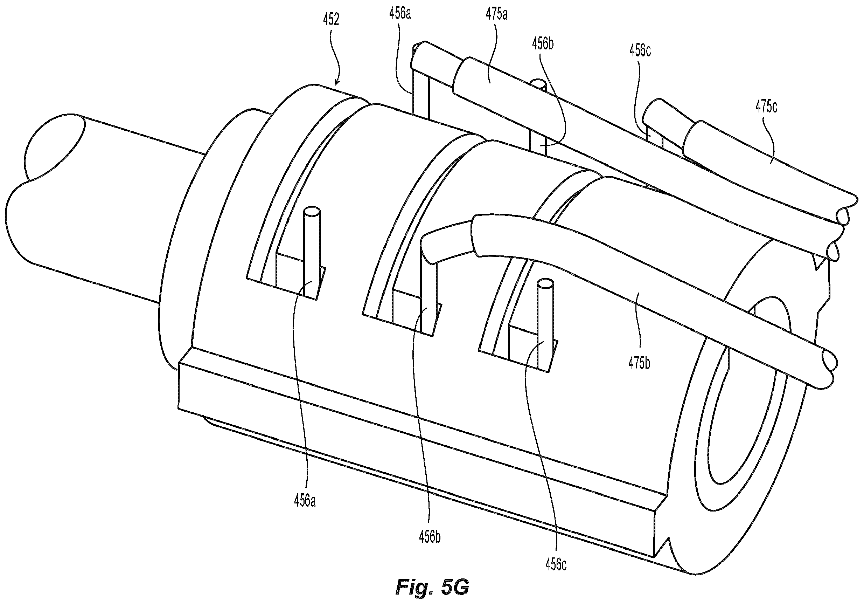

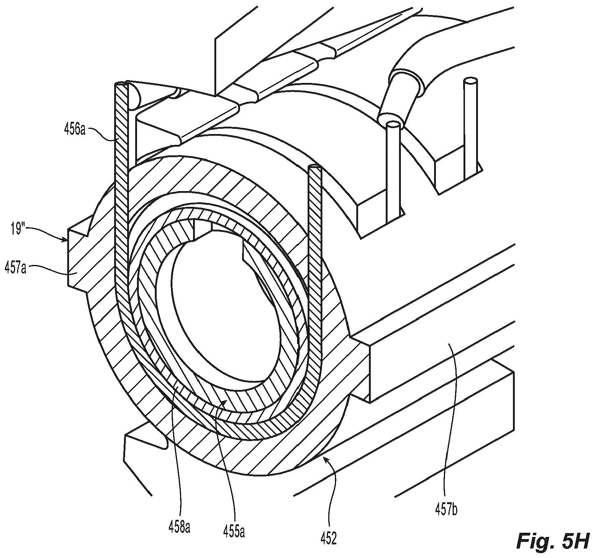

Once the bobbin 452 is engaged atop the CRC 450 and the contact wires 456a-456c are locked therein, corresponding jumper wires 475a-475c are soldered or otherwise engaged to respective contact wires 456a-456c. The jumper wires, e.g., jumper wire 475a, may be engaged to either leg of the respective contact wire, e.g., contact wire 456a (FIG. 5G). The bobbin 452 is then secured into the cavity 19' of the proximal shaft connector 19 and is constrained therein by one or more mechanically inter-engaging components. For example, bobbin 452 may include elongated flanges 457a, 457b disposed along an outer peripheral surface thereof which mechanically cooperate with corresponding channels 19" defined along the inner surface of cavity 19' of proximal shaft connector 19 (FIG. 5H). As a result thereof, the bobbin 452 cannot spin relative to the proximal shaft connector 19 of shaft 12b. The bobbin 452 also prevents the contact wires 456a-456c from spinning, however, the CRC 450 and the cable 210 can rotate freely therein.

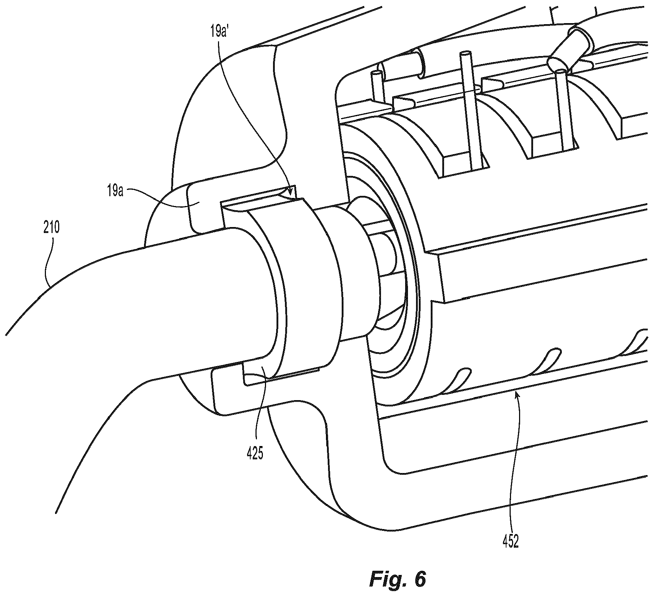

FIG. 6 show the strain relief 425 engaged to the cable 210 and mechanically secured within flange 19a of the proximal shaft connector 19. More particularly, the strain relief 425 is crimped to the outer jacket of the cable 210 and mechanically secured within a strain cavity 19a' defined in the flange 19a of the proximal shaft connector 19. Strain relief 425 prevents relative movement of the cable 210 and the proximal shaft connector 19 reducing tension on the leads 210a-210c contained therein while at the same time allowing rotation of the cable 210 relative to the proximal shaft connector 19. As can be appreciated this enables the surgeon to freely wield the forceps 10 without tangling or concerns about unnecessarily tensioning the cable 210 or internal electrical connections.

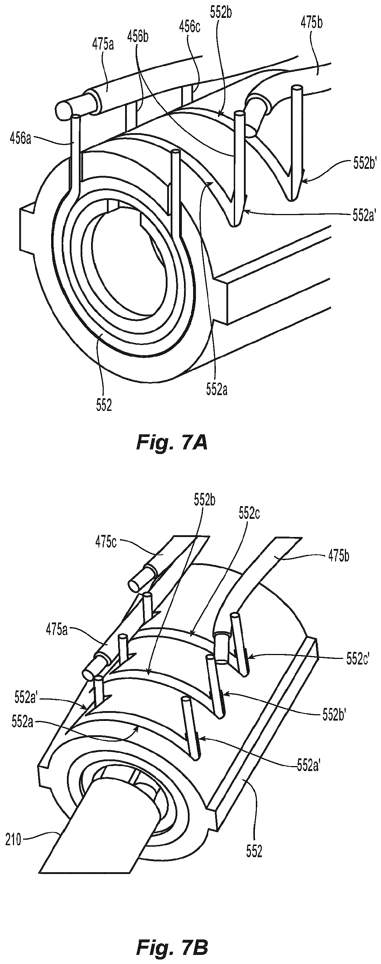

FIGS. 7A and 7B show an alternate embodiment of the locking bobbin 552 for use with the CRC 450. More particularly, bobbin 552 includes angled locking recesses 552a'-552c'defined within the corresponding slots 552a-552c. Once the wires 456a-456c are engaged through both the upper slots 552a-552c and the lower slots (not shown), respectively, the bobbin 552 is moved relative to the CRC 450, e.g., slid proximally, to lock the contact wires 456a-456c in place in electrical engagement with the CRC 450 (FIGS. 7A and 7B). Upon proximal movement, the contact wires 456a-456c engage locking recesses 552a'-552c' defined within the corresponding slots 552a-552c of bobbin 552.

The various embodiments disclosed herein may also be configured to work with robotic surgical systems and what is commonly referred to as "Telesurgery." Such systems employ various robotic elements to assist the clinician and allow remote operation (or partial remote operation) of surgical instrumentation. Various robotic arms, gears, cams, pulleys, electric and mechanical motors, etc. may be employed for this purpose and may be designed with a robotic surgical system to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of clinicians may prep the patient for surgery and configure the robotic surgical system with one or more of the instruments disclosed herein while another clinician (or group of clinicians) remotely controls the instruments via the robotic surgical system. As can be appreciated, a highly skilled clinician may perform multiple operations in multiple locations without leaving his/her remote console which can be both economically advantageous and a benefit to the patient or a series of patients.

For a detailed description of exemplary medical work stations and/or components thereof, reference may be made to U.S. Patent Application Publication No. 2012/0116416, and PCT Application Publication No. WO2016/025132, the entire contents of each of which are incorporated by reference herein.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be affected by one skilled in the art without departing from the scope or spirit of the disclosure. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. For example, the knife body and tube do not necessarily have to be made from the exact same materials. Similar materials, or any two materials that can be welded together to allow for a durable weld joint could be used.

The invention may be described by reference to the following numbered paragraphs:-
1. A coupling for an electrosurgical cable of a surgical instrument, comprising:
   a plurality of insulative contact collars arranged in a stack-like manner, each of the plurality of contact collars including a flange configured to support a corresponding plurality of contact rings thereon, the plurality of contact rings configured for electrical engagement with a corresponding plurality of electrical leads disposed through an electrosurgical cable;
   a plurality of contact wires, each contact wire configured to engage a corresponding contact ring at one end thereof and a jumper wire at another end thereof, the jumper wires adapted to electrical couple to an activation switch of a surgical instrument; and
   a locking bobbin configured to at least partially envelop the plurality of insulative contact collars and lock the plurality of contact wires in secure electrical engagement with the plurality of contact rings, the locking bobbin including at least one mechanical interface that cooperates with a corresponding mechanical interface disposed within the surgical instrument to secure the locking bobbin therein.
2. The coupling for an electrosurgical cable of a surgical instrument according to paragraph 1, wherein the plurality of collars defines a passageway therethrough for receiving the electrical leads from the electrosurgical cable.
3. The coupling for an electrosurgical cable of a surgical instrument according to paragraph 1, wherein the contact wires are U-shaped and include two opposing legs, the opposing legs of each contact wire configured to engage the opposing sides of each contact ring.
4. The coupling for an electrosurgical cable of a surgical instrument according to paragraph 1, wherein the flanges of each contact collar of the plurality of contact collars is recessed relative to an outer peripheral surface of each contact collar of the plurality of contact collars such that, when electrically engaged with the corresponding plurality of contact wires, the plurality of contact wires lie flush with the outer peripheral surface of the plurality of contact collars.
5. The coupling for an electrosurgical cable of a surgical instrument according to paragraph 1, wherein the locking bobbin includes a corresponding plurality of slots defined therein configured to receive the plurality of contact wires therethrough.
6. The coupling for an electrosurgical cable of a surgical instrument according to paragraph 5, wherein each slot of the plurality of slots includes a recess configured to lock the respective contact wire therein.
7. The coupling for an electrosurgical cable of a surgical instrument according to paragraph 6, wherein each recess of each slot of the plurality of slots is configured to lock the respective contact wire therein upon movement of the locking bobbin relative to the plurality of contact collars from a first position to a second position.
8. The coupling for an electrosurgical cable of a surgical instrument according to paragraph 1, wherein each lead of the plurality of electrical leads disposed through the electrosurgical cable is soldered to each corresponding contact ring.
9. The coupling for an electrosurgical cable of a surgical instrument according to paragraph 1, wherein each contact wire of the plurality of contact wires is soldered to a corresponding jumper wire.
10. The coupling for an electrosurgical cable of a surgical instrument according to paragraph 7, wherein each recess of each slot of the plurality of slots is angled relative to each slot of the plurality of slots to lock the respective contact wire therein upon movement of the locking bobbin relative to the plurality of contact collars from a first position to a second position.
11. The coupling for an electrosurgical cable of a surgical instrument according to paragraph 1, wherein the locking bobbin includes at least one elongated flange that cooperates with a corresponding elongated channel disposed within the surgical instrument to secure the locking bobbin therein.
12. A surgical instrument, comprising:
   an continuous rotation coupling (CRC) including:
      a plurality of insulative contact collars arranged in a stack-like manner, each of the plurality of contact collars including a flange configured to support a corresponding plurality of contact rings thereon, the plurality of contact rings configured for electrical engagement with a corresponding plurality of electrical leads disposed through an electrosurgical cable;
      a plurality of contact wires, each contact wire configured to engage a corresponding contact ring at one end thereof and a jumper wire at another end thereof, the jumper wires adapted to electrical couple to an activation switch of a surgical instrument;
   a locking bobbin configured to at least partially envelop the CRC to lock the plurality of contact wires in secure electrical engagement with the plurality of contact rings, the locking bobbin including at least one mechanical interface that cooperates with a corresponding mechanical interface disposed within the surgical instrument to secure the locking bobbin therein, the locking bobbin preventing the plurality of contact wires from rotating within the surgical instrument while permitting the plurality of contact collars, the plurality of contact rings and the electrosurgical cable to rotate relative thereto; and
   a strain relief disposed about the electrical cable, the strain relief configured to seat within a strain cavity defined within a proximal flange of the surgical instrument, the strain relief configured to allow rotation of the electrosurgical cable relative to the surgical instrument but prevent translation of the electrosurgical cable relative to the surgical instrument.
13. The coupling for an electrosurgical cable of a surgical instrument according to paragraph 12, wherein the strain relief is crimped to the electrosurgical cable.
14. The coupling for an electrosurgical cable of a surgical instrument according to paragraph 12, wherein the plurality of collars defines a passageway therethrough for receiving the electrical leads from the electrosurgical cable.
15. The coupling for an electrosurgical cable of a surgical instrument according to paragraph 12, wherein the contact wires are U-shaped and include two opposing legs, the opposing legs of each contact wire configured to engage the opposing sides of each contact ring.
16. The coupling for an electrosurgical cable of a surgical instrument according to paragraph 12, wherein the flanges of each contact collar of the plurality of contact collars is recessed relative to an outer peripheral surface of each contact collar of the plurality of contact collars such that, when electrically engaged with the corresponding plurality of contact wires, the plurality of contact wires lie flush with the outer peripheral surface of the plurality of contact collars.
17. The coupling for an electrosurgical cable of a surgical instrument according to paragraph 12, wherein the locking bobbin includes a corresponding plurality of slots defined therein configured to receive the plurality of contact wires therethrough.
18. The coupling for an electrosurgical cable of a surgical instrument according to paragraph 17, wherein each slot of the plurality of slots includes a recess configured to lock the respective contact wire therein.
19. The coupling for an electrosurgical cable of a surgical instrument according to paragraph 18, wherein each recess of each slot of the plurality of slots is configured to lock the respective contact wire therein upon movement of the locking bobbin relative to the plurality of contact collars from a first position to a second position.
20. The coupling for an electrosurgical cable of a surgical instrument according to paragraph 12, wherein each lead of the plurality of electrical leads disposed through the electrosurgical cable is soldered to each corresponding contact ring.
21. The coupling for an electrosurgical cable of a surgical instrument according to paragraph 12, wherein each contact wire of the plurality of contact wires is soldered to a corresponding jumper wire.
22. The coupling for an electrosurgical cable of a surgical instrument according to paragraph 18, wherein each recess of each slot of the plurality of slots is angled relative to each slot of the plurality of slots to lock the respective contact wire therein upon movement of the locking bobbin relative to the plurality of contact collars from a first position to a second position.
23. The coupling for an electrosurgical cable of a surgical instrument according to paragraph 12, wherein the locking bobbin includes at least one elongated flange that cooperates with a corresponding elongated channel disposed within the surgical instrument to secure the locking bobbin therein.

## Claims

1. A coupling for an electrosurgical cable of a surgical instrument, comprising:
a plurality of insulative contact collars arranged in a stack-like manner, each of the plurality of contact collars including a flange configured to support a corresponding plurality of contact rings thereon, the plurality of contact rings configured for electrical engagement with a corresponding plurality of electrical leads disposed through an electrosurgical cable;
a plurality of contact wires, each contact wire configured to engage a corresponding contact ring at one end thereof and a jumper wire at another end thereof, the jumper wires adapted to electrical couple to an activation switch of a surgical instrument; and
a locking bobbin configured to at least partially envelop the plurality of insulative contact collars and lock the plurality of contact wires in secure electrical engagement with the plurality of contact rings, the locking bobbin including at least one mechanical interface that cooperates with a corresponding mechanical interface disposed within the surgical instrument to secure the locking bobbin therein.

2. The coupling for an electrosurgical cable of a surgical instrument according to claim 1, wherein the plurality of collars defines a passageway therethrough for receiving the electrical leads from the electrosurgical cable.

3. The coupling for an electrosurgical cable of a surgical instrument according to claim 1 or 2, wherein the contact wires are U-shaped and include two opposing legs, the opposing legs of each contact wire configured to engage the opposing sides of each contact ring.

4. The coupling for an electrosurgical cable of a surgical instrument according to claim 1, 2or 3 wherein the flanges of each contact collar of the plurality of contact collars is recessed relative to an outer peripheral surface of each contact collar of the plurality of contact collars such that, when electrically engaged with the corresponding plurality of contact wires, the plurality of contact wires lie flush with the outer peripheral surface of the plurality of contact collars.

5. The coupling for an electrosurgical cable of a surgical instrument according to any preceding claim, wherein the locking bobbin includes a corresponding plurality of slots defined therein configured to receive the plurality of contact wires therethrough.

6. The coupling for an electrosurgical cable of a surgical instrument according to claim 5, wherein each slot of the plurality of slots includes a recess configured to lock the respective contact wire therein.

7. The coupling for an electrosurgical cable of a surgical instrument according to claim 6, wherein each recess of each slot of the plurality of slots is configured to lock the respective contact wire therein upon movement of the locking bobbin relative to the plurality of contact collars from a first position to a second position.

8. The coupling for an electrosurgical cable of a surgical instrument according to any preceding claim, wherein each lead of the plurality of electrical leads disposed through the electrosurgical cable is soldered to each corresponding contact ring.

9. The coupling for an electrosurgical cable of a surgical instrument according to any preceding claim, wherein each contact wire of the plurality of contact wires is soldered to a corresponding jumper wire.

10. The coupling for an electrosurgical cable of a surgical instrument according to any preceding claim, wherein each recess of each slot of the plurality of slots is angled relative to each slot of the plurality of slots to lock the respective contact wire therein upon movement of the locking bobbin relative to the plurality of contact collars from a first position to a second position.

11. The coupling for an electrosurgical cable of a surgical instrument according to any preceding claim, wherein the locking bobbin includes at least one elongated flange that cooperates with a corresponding elongated channel disposed within the surgical instrument to secure the locking bobbin therein.

12. A surgical instrument, comprising:
a coupling as claimed in any preceding claim :
and
a strain relief disposed about the electrosurgical cable, the strain relief configured to seat within a strain cavity defined within a proximal flange of the surgical instrument, the strain relief configured to allow rotation of the electrosurgical cable relative to the surgical instrument but prevent translation of the electrosurgical cable relative to the surgical instrument.

13. The coupling for an electrosurgical cable of a surgical instrument according to claim 12, wherein the strain relief is crimped to the electrosurgical cable.
